# EUROPEAN PATENT APPLICATION

(11) **EP 4 480 402 A1**
(43) Date of publication of application: **25.12.2024**
(21) Application number: 23184064.6
(22) Date of filing: 07.07.2023
(51) Int. Cl.: A61B 5/024, A61B 5/026, A61B 5/0295, A61B 5/00

(54) **DEVICE, SYSTEM AND METHOD FOR DETERMINING A BLOOD PERFUSION VALUE OF A SUBJECT**

(30) Priority: 23.06.2023 US 202363522740 P
(71) Applicant: Koninklijke Philips N.V., 5656 AG Eindhoven (NL)
(72) Inventor: VERKRUIJSSE, Willem, Eindhoven (NL); JAFFE, Mike, Eindhoven (NL)
(74) Representative: Philips Intellectual Property & Standards

(57) **Abstract**

The present invention relates to device, system and method for determining a blood perfusion value of a subject as well as an appropriate sensor. The device comprises circuitry configured to: obtain a photoplethysmography, PPG, signal acquired by a sensor including a light emitter and a light detector at a measurement location of the subject; determine a modulation depth of the obtained PPG signal; and determine the blood perfusion value of the subject by normalizing, based on measurement-related information indicative of the length of the light path of the light through the subject's tissue from the light emitter to the light detector and/or of the tissue volume of the subject's tissue through which the light travels from the light emitter to the light detector, the determined modulation depth or a perfusion index determined from the modulation depth.

## Description

### FIELD OF THE INVENTION

The present invention relates to a device, system and method for determining a blood perfusion value of a subject as well as a sensor for use in such a system.

### BACKGROUND OF THE INVENTION

Pulse oximeters are widely used to indirectly monitor the oxygen saturation (SpO₂) of a subject's (e.g. patient's or other person's) blood and changes in blood volume in the skin, producing a photoplethysmogram (also called "photoplethysmography signal" or "PPG signal") that may be further processed into other measurements. A pulse oximeter generally comprises a sensor including a light emitter and a light detector. In a transmissive pulse oximetry approach, the sensor is placed on a thin part of the subject's body, usually a fingertip, earlobe or nasal alar. The light emitter passes two (or more) wavelengths of light through the body part (also called "anatomical object" at the measurement location) to the light detector, which measures the changing transmittance at each of the wavelengths, allowing it to determine the absorbances due to the pulsing arterial blood. In a reflectance pulse oximetry approach, light reflected from the body part in response to the light emission from the light emitter is detected by the light detector to obtain the PPG signal.

From the PPG signal, its modulation depth and a perfusion index (PI), which is generally proportional to the modulation depth, can be determined. The absolute value of the PI and changes in it have been used clinically since it was first introduced in pulse oximeters. Most of the studies characterizing the expected values of PI and changes of it have been made at the finger, a peripheral site (as one example of a "measurement location"). With the greater use of central sites (other examples of "measurement locations") with different optical, physiologic and anatomic characteristics such as the nasal alar and earlobe, clinicians are starting to look to those sites for pulse oximetry and as such looking at and trying to interpret the absolute value of and changes of PI relative to values which they have grown familiar. Clinicians may often initially seek to obtain an SpOz and PI value from a peripheral site and find it noisy or not available due to low perfusion (e.g. from cold, shock, etc.) or other factors and seek to find another site with more reliable SpO₂ and higher PI. However, this may lead to significantly different values in the PI for the different sites.

Hence, PI values are difficult to compare from one subject to another and from one site to another on the same subject or on different subjects. Although PI is still widely considered an individual site-specific metric for perfusion, PI values cannot be reliably used to quantify the overall perfusion of a subject, regardless of where it is measured.

### SUMMARY OF THE INVENTION

It is an object of the present invention to provide a device, system and method allowing to determine a more meaningful measure of perfusion of a subject. It is a further object of the present invention to provide a sensor that may be used in a system for determining such a more meaningful measure of perfusion of the subject.

In a first aspect of the present invention a device for determining a blood perfusion value of a subject is presented comprising circuitry configured to:
- obtain a photoplethysmography, PPG, signal acquired by a sensor including a light emitter and a light detector at a measurement location of the subject;
- determine a modulation depth of the obtained PPG signal; and
- determine the blood perfusion value of the subject by normalizing, based on measurement-related information indicative of the length of the light path of the light through the subject's tissue from the light emitter to the light detector and/or of the tissue volume of the subject's tissue through which the light travels from the light emitter to the light detector, the determined modulation depth or a perfusion index determined from the modulation depth.

In a further aspect of the present invention a system for determining a blood perfusion value of a subject is presented, the system comprising:
- a sensor including a light emitter and a light detector configured to acquire a PPG signal at a measurement location of the subject; and
- a device as disclosed herein for determining the blood perfusion value from the acquired PPG signal.

In a still further aspect of the present invention a sensor for acquiring a PPG signal at a measurement location of a subject is presented, the sensor comprising:
- a light emitter configured to emit light towards the measurement location;
- a light detector configured to detect light reflected from or transmitted through the subject's tissue at the measurement location; and
- a distance information element configured to measure the distance between the light emitter and the light detector or to determine distance-related information allowing to determine the distance between the light emitter and the light detector.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the recognition that the distance between the light sensor and the light detector has an effect on the modulation depth which thus cannot be interpreted as representative of the tissue perfusion alone, and that by accounting for these effects a value can be computed in which these effects are minimized, resulting in a value that is more representative of tissue perfusion. More generally, it has been found that the optical path length, i.e., the length of the path that the light travels from the light emitter through the tissue to the light detector affects the PI. In the same or similar manner, this optical path length affects the modulation depth of the PPG signal. A similar effect has the tissue volume through which the light travels from the light emitter to the light detector.

It is thus proposed to normalize the PI or modulation depth and thus account for the effect these factors have on the modulation depth (and PI) based on measurement-related information that is indicative of the length of the light path of the light through the subject's tissue from the light emitter to the light detector and/or of the tissue volume of the subject's tissue through which the light travels from the light emitter to the light detector. The measurement-related information may directly be this length of the light path or this tissue volume, but various other data may be used as measurement-related information that have an influence on the PI and modulation path.

Thus, according to the present invention, a new and more beneficial metric related to blood perfusion is presented, which is herein called blood perfusion value and which may be used as a more "absolute" measure of blood perfusion than the modulation depth of the PPG signal or than a perfusion index. Clinicians may thus e.g. be able to better compare the blood perfusion value from alar sensors with blood perfusion value from finger clips and use this as a measure of centralization. Doing this with PI values would be much less precise. In addition to obtaining a measure of centralization within one subject, the more 'absolute' measure of perfusion may also serve to more accurately compare values between subjects.

In the context of the present invention, "normalizing" the PI or the modulation depth shall be understood as "modifying" the PI or the modulation depth by use of certain information or subjecting it to a function using the certain information. This certain information is related to the length of the light path and/or of the tissue volume through which the light travels. Such information is not taken into account in the conventional computation of PI or modulation depth.

According to an embodiment, the circuitry (e.g. a processor, computer or other entity implemented in software and/or hardware) is configured to
- subject the determined modulation depth or perfusion index to a normalization function including one or more normalization values and
- determine the one or more normalization values from the measurement-related information.

The normalization function may be a scaling function using a normalization factor. In other embodiment, a more complex normalization function may be applied. For instance, according to an embodiment the circuitry is configured to use a look-up table or a normalization model or a linear, exponential, logarithmic or polynomial normalization function for normalizing the determined modulation depth or perfusion index. The normalization function may be determined empirically or analytically. For instance, if empirical data is not present, e.g. in an application where true values for perfusion are not easy to measure or are not reliable, an analytically determined normalization functions obtained from a model may be used. For instance, results from models in which random numbers are generated to simulate light distributions, or functions determined with Monte Carlo techniques might be used as empirical or analytical functions.

In an embodiment, the circuitry is configured to use subject-related data as measurement-related information or to derive measurement-related information from subject-related data, the subject-related data including one or more of:
- the subject's body mass index (BMI),
- the subject's weight,
- the subject's size,
- the subject's gender,
- the subject's race or ethnicity,
- the subject's health condition or diagnosis (e.g. a clinical diagnosis, such as a vascular disease that has altered the distribution of vessels in the anatomical location),
- the subject's physiological status (e.g. finger temperature, blood pressure values, fluid status, cardiac output, etc.),
- the subject's blood pressure,
- the subject's fluid status,
- the orthostatic position of the measurement location (e.g. if the finger elevated with respect to the heart), and
- the thickness of an anatomical object at the measurement location, in particular the thickness of a finger, the earlobe or the alar of the subject.

From such subject-related data information on the distance between the light emitter and the light detector can be inferred. For instance, if the subject has a higher BMI or weight, it is inferred that the subject's finger is thicker so that the distance is larger compared to a subject having a lower BMI or weight. This information may then be used for adapting the normalization of the modulation depth or PI accordingly.

In another embodiment, the circuitry is configured to use location-related data as measurement-related information or to derive measurement-related information from location-related data, the location-related data including one or more of:
- the location of measurement of the PPG signal,
- the type of anatomical object at the measurement location,
- the type of sensor used for acquiring the PPG signal,
- the temperature and/or shunt status of the tissue at the measurement location,
- the geometry of light transmission through an anatomical object at the measurement location from the light emitter to the light detector,
- the distance between the light emitter and the light detector of the sensor,
- the total attenuation of the light through the anatomical object at the measurement location from the light emitter to the light detector.

From such location-related data information on the distance between the light emitter and the light detector can be inferred as well. For instance, if the type of anatomical object is a finger, it is inferred that the distance is larger compared to when the type of anatomical object is the nasal alar or earlobe. The type of anatomical object may be inferred from the type of sensor used or from an input by the user or subject.

The circuitry may further be configured to
- determine the distance and/or tissue volume between the light emitter and the light detector from the obtained measurement-related information, and
- normalize the determined perfusion index by multiplying it by a normalization factor that is larger the smaller the determined distance and/or the determined tissue volume is.

The distance or tissue volume may be either known empirically, for instance from an average distance measured for the same type of patient or may be determined from subject-related data or located-related data. Further, it may be inferred from another measurement, such as the angle of the two clip parts of a finger clip. Still further, various direct measurement methods may be used to determine the distance, such as electronic distance measurement methods (EDM) using e.g. time of flight, interference, or other techniques to measure distance. Even the total attenuation of the light through the anatomical object from the light emitter to the light detector may be used as useful information to determine the distance.

In another embodiment, the circuitry is configured to obtain the measurement-related information from a measurement element, a database or a user interface. A measurement element, as mentioned above, may directly measure the measurement-related information or data from which measurement-related information can be inferred. A database may provide a list of measurement-related information in relation to other measured parameters (e.g. a list of distance between light emitter and light detector for different BMI, or different anatomical objects, etc.). A user interface, e.g. a keypad or touchscreen, may be used to enter the measurement-related information or data from which measurement-related information can be inferred.

The circuitry may further be configured in an embodiment to determine the perfusion index of the subject as a ratio of the AC component to the DC component of the PPG signal or as a ratio of a weighted average of the AC component to the DC component of the PPG signal at different wavelengths. The ratio may e.g. be a weighted average of the red and infrared (IR) (or other used wavelengths) PPG signals.

The circuitry may further be configured to
- determine a blood perfusion value for different PPG signals acquired at different measurement locations of the subject and/or acquired from different subjects, and
- compare and/or combine the determined blood perfusion values.
The blood perfusion values determined for different subjects can be compared or combined much better than modulation depth values or PI values determined for different subjects and thus allow to better judge the health status of a subject compared to other subjects. In addition, blood perfusion values determined for a single subject at different measurement locations and/or at different times and/or using different sensors may be much better compared or combined.

In another embodiment the circuitry is configured to determine, based on the determined blood perfusion value, if the measurement location and/or arrangement of a sensor used for acquiring the PPG signal should be modified. Since the blood perfusion value is more meaningful and objective than PI, any consequences decided or recommended based on the blood perfusion value will be more reasonable and accurate.

As mentioned above, the system according to the present invention comprises, in addition to the device for determining a blood perfusion value of a subject described above, a sensor including a light emitter and a light detector configured to acquire a PPG signal at a measurement location of the subject. Further, the system may comprise a distance information element configured to measure the distance between the light emitter and the light detector or to determine distance-related information allowing to determine the distance between the light emitter and the light detector.

There are various embodiments how the distance information element can be implemented. These embodiments include:
- a distance measurement element configured to measure the distance or optical path length between the light emitter and the light detector,
- an angle measurement element configured to measure the angle between the light emitter and the light detector of the sensor configured as clip,
- a distance estimation element configured to estimate the distance or optical path length between the light emitter and the light detector based on subject-related data and/or location-related data, and
- a pressure measurement element configured to measure the pressure or force applied on the anatomical object at the measurement location by the sensor configured as clip.

For instance, a time of flight (TOF) sensor may be used to simultaneously measure a PPG signal and a distance travelled by the light, which may give an estimate of the distance or path length between light emitter and light detector. Other options to measure distance may be to use the DC attenuation of the light, EDM (Electronic distance measurement), and empirical (based on gender, age, BMI, etc.).

Further, as mentioned above, a sensor is generally used acquiring a PPG signal at a measurement location of a subject. A sensor generally comprises:
- a light emitter configured to emit light towards the measurement location; and
- a light detector configured to detect light reflected from or transmitted through the subject's tissue at the measurement location.

A more advanced version of a sensor according to the present invention additionally comprises a distance information element configured to measure the distance between the light emitter and the light detector or to determine distance-related information allowing to determine the distance between the light emitter and the light detector. The distance information element may be configured as explained above in the context of an embodiment of the system according to the present invention.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention;
Fig. 2A shows a side view of a first embodiment of a sensor according to the present invention in open state;
Fig. 2B shows a side view of the sensor shown in Fig. 2A in closed state;
Fig. 2C shows a front view of the sensor shown in Fig. 2A in closed state;
Fig. 3 shows a schematic diagram of an embodiment of a device according to the present invention;
Fig. 4 shows a schematic diagram of an embodiment of a method according to the present invention;
Fig. 5 shows a schematic diagram of another embodiment of a system according to the present invention;
Fig. 6 shows a side view of a second embodiment of a sensor according to the present invention;
Fig. 7 shows a side view of a third embodiment of a sensor according to the present invention; and
Fig. 8 shows a side view of a fourth embodiment of a sensor according to the present invention.

### DETAILED DESCRIPTION OF EMBODIMENTS

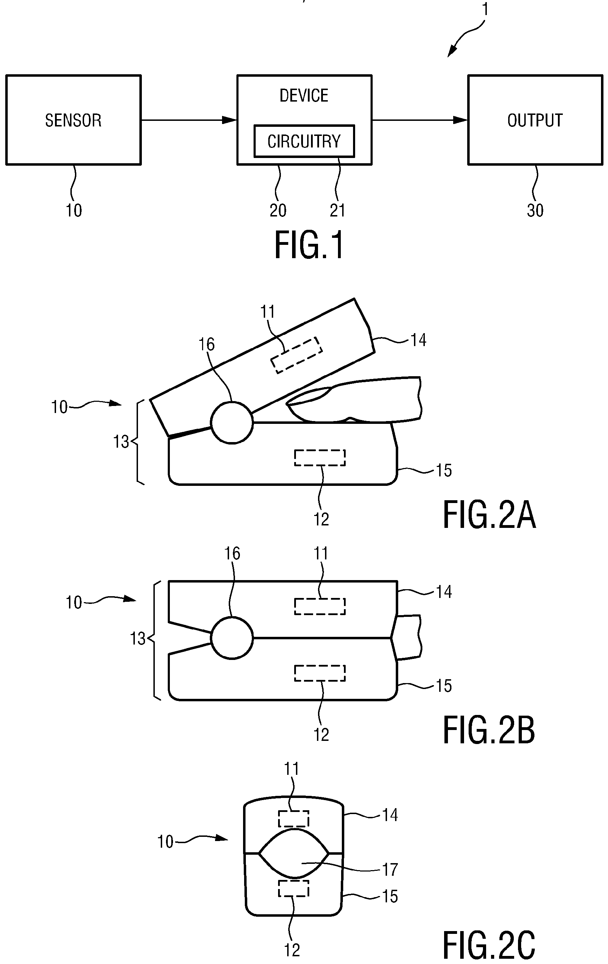

Fig. 1 shows a schematic diagram of an embodiment of a system 1 for determining a blood perfusion value of a subject. The system 1 comprises a sensor 10 including a light emitter 11 and a light detector 12 configured to acquire a photoplethysmography (PPG) signal at a measurement location of the subject. The system 1 further comprises a device 20 for determining the blood perfusion value from the acquired PPG signal as will be explained in more detail below. Optionally, the system 1 may further comprise an output interface 30 configured to issue the determined blood perfusion value.

Fig. 2 shows different views of the sensor 10. In this exemplary embodiment the sensor 10 is configured substantially in the same manner as a conventional pulse oximeter in the form of a finger clip that can be clipped at the subject's finger. Fig. 2A shows the sensor 10 in open state, Fig. 2B shows the sensor 10 in closed state, and Fig. 2C shows a front view of the sensor in closed state. The sensor 10 has a housing 13 formed by two housing portions 14, 15 that are hinged together by a swivel joint 16. Between the two housing portions 14, 15 a measurement location 17 (also called measurement area) is formed.

Light is emitted by the light emitter 11 towards the measurement location, which is the part (in this example the fingertip) of the subject's anatomical object (in this example the finger) that is arranged in the measurement area 17. The light detector 12 detects light that has, after emission from the light emitter 11, transmitted through the subject's tissue (i.e., the fingertip) at the measurement location 17.

In other embodiments the sensor 10 may be configured for arrangement at other anatomical objects, such as the ear lobe or the nasal alar. It may be configured as clip for clipping it to the anatomical object, as shown in Fig. 2, or as wearable, e.g. in the form of or integrated into a smartwatch, belt or other means that can be mounted (e.g. taped) to the subject's body. Particularly in sensors formed as wearables, the light detector 12 may be configured to detect light that has, after emission from the light emitter 11, been reflected from the subject's tissue, in particular the subject's skin.

The light emitter 11 may comprises an LED unit for emitting light in the visible wavelength range, e.g. an RGB LED unit, or a single LED emitting light in a single wavelength channel, e.g. red light. For detecting certain vital signs, such as oxygen saturation (SpOz), the light emitter 11 emits light in at least two wavelength channels, preferably red light and near-infrared light, either simultaneously or alternately.

The light detector 12 may comprises a single photosensor unit that is sensitive to light in a broad wavelength range or only in the wavelength range of interest or in which light is emitted by the light emitter (e.g. red light only or red and near-infrared light). In other embodiments separate photodiodes may be used which are each sensitive for light in different wavelength channels (e.g. one photodiode for red light and another photodiode for near-infrared light).

From the detected light one or more PPG signals are derived. A PPG signal represents the intensity of the detected light over time in a wavelength channel of interest. For instance, two PPG signals in the red and near-infrared wavelength channels may be derived from the detected light. This may be done by the light detector 12, which may comprise processing circuitry to process the detected light accordingly. In other embodiments this processing may be done in the device 20.

The device 20 may be implemented by respective units or circuitry 21, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 20, which may execute a computer program that may be stored in a memory that is accessed by the processor.

The device 20 may be a separate device or may be combined with the sensor 10, e.g. may be integrated into the housing 13 of the sensor 10. The PPG signals acquired by the sensor may be provided to the device 20 through a wired or wireless connection.

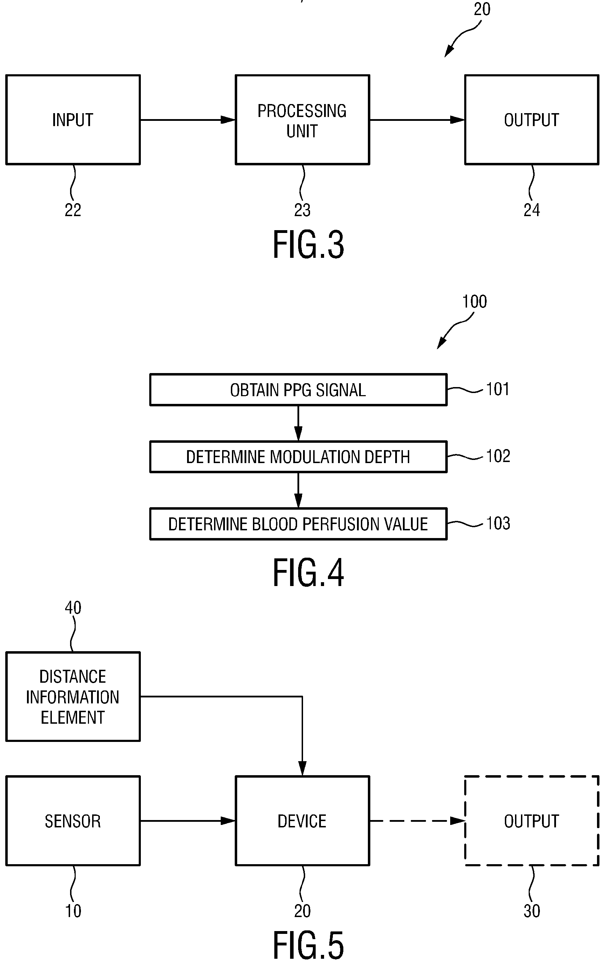

Fig. 3 shows a schematic diagram of an embodiment of a device 20 for determining the blood perfusion value according to the present invention. In this embodiment, the device 20 comprises an input 22 configured to obtain the PPG signal(s) from the sensor 10. The input 22 may be directly coupled or connected to the sensor 10 or may obtain (i.e. retrieve or receive) the PPG signal(s) from a storage, buffer, network, or bus, etc. The input 22 may thus e.g. be (wired or wireless) communication interface or data interface, such as a Bluetooth interface, WiFi interface, LAN interface, HDMI interface, direct cable connect, or any other suitable interface allowing signal transfer to the device 20.

The device 20 further comprises a processing unit 23 configured to determine the blood perfusion value. The processing unit 23 may be any kind of means configured to process the PPG signal(s) and determine the blood perfusion value there from. It may be implemented in software and/or hardware, e.g. as a programmed processor or computer or app on a user device such as a smartphone, smartwatch, tablet, laptop, PC, workstation, etc.

The device 20 further comprises an output 24 configured to output the determined blood perfusion value. The output 24 may generally be any interface that provides the determined blood perfusion value, e.g. transmits it to another device or provides it for retrieval by another device (e.g. a smartphone, computer, tablet, etc.). It may thus generally be any (wired or wireless) communication or data interface.

The input 22, the processing unit 23 and the output 24 may together form the circuitry 21 of the device 20.

The optional output interface 30 may generally be any means that outputs the determined blood perfusion value in visual or audible form, e.g. in text form, as image or diagram, as sound or spoken words, etc. For instance, the output interface 30 may be a display, a loudspeaker, a touchscreen, a computer monitor, the screen of a smartphone or tablet, etc. In an embodiment, the output interface 30 may be a separate device or may be combined with the sensor 10 or the device 20, e.g. may be integrated into the housing 13 of the sensor 10, for instance in the form of a display within or on top of a surface of one of the housing portions 14, 15.

Fig. 4 shows a schematic diagram of an embodiment of a method 100 for determining the blood perfusion value according to the present invention. The steps of the method may be carried out by the device 20, wherein the main steps of the method are carried out by the processing unit 23. The method may e.g. be implemented as computer program running on a computer or processor.

In a first step 101 a PPG signal acquired by the sensor 10 is obtained. In a second step 102 a modulation depth of the obtained PPG signal is determined. In a third step 103 the blood perfusion value of the subject is determined by normalizing, based on measurement-related information indicative of the length of the light path of the light through the subject's tissue from the light emitter to the light detector and/or of the tissue volume of the subject's tissue through which the light travels from the light emitter to the light detector, the determined modulation depth or a perfusion index determined from the modulation depth. In this way, a novel metric (called blood perfusion value herein) is introduced by the present invention, which may be derived by normalization from another (existing) metric (the perfusion index PI) or from the modulation depth of PPG signal. PI itself is not exactly defined, but there are different ways to determine PI. For instance, different manufacturers of pulse oximeters may use different algorithms to calculate PI from a PPG signal. The present invention is, however, independent of the way in which PI is calculated since, as one option, PI is normalized to determine the blood perfusion value. In another option, PI is not used at all, but modulation depth is normalized to obtain the blood perfusion value.

In some embodiments, the PPG modulation depth may be used as PI, sometimes as a weighted average of the PPG modulation depths e.g. at the red and IR wavelengths. Furthermore, generally the same normalization may be applied for normalizing any PI or the PPG modulation depth.

As explained above, conventionally clinicians often try to compare PI values obtained from different sites and/or obtained from different subjects. This comparison can be problematic given the different tissue thicknesses and the associated different optical paths between the source and detectors, e.g. between the fingers or even amongst fingers, earlobes, nasal alar and forehead. The use of the PI as currently shown on patient monitors is not considered an "absolute" measure of perfusion, despite its name. Rather, it is perceived as an individual measure of perfusion. It can be used to find a good spot for pulse oximetry, e.g. by finding a spot with high PI, and it may be used to monitor changes in PI. However, PI depends on many factors, other than the actual perfusion.

To make the PI less dependent on the saturation of the arterial blood, a weighted average may be calculated from the red and near-infrared PPG wavelengths. However, saturation is not the only parameter that influences PI; the optical path (e.g. due to the thickness of a finger or ear lobe) is a strong determinant of the measured PI as well. The rather limited use of PI as an absolute measure for perfusion is thus likely due to the fact that the reported PI does not take the variations in optical path, in particular the length of the optical path, into account.

In practice, this means that for finger clip probes, the PI tends to be larger for thicker fingers (e.g. middle finger, large optical path), and smaller for thinner fingers (e.g. pinky, smaller optical path) on the same subject. Further, subjects with thicker fingers tend to have larger PI values than subjects with smaller fingers. This correlation has been proven by studies with a plurality of subjects. Averaging all the PI values for each finger showed an almost linear relationship between average finger size and average PI.

The present invention thus proposes to normalize PI or modulation depth to remove the dependency on the optical path. This will result a more useful physiological metric called blood perfusion value herein. The non-thumb fingers on average show a large dynamic range (from 1.1 to 2.7) of PI. The linear dependency on finger width may be exploited by embodiments of the present invention and nominal finger sizes may be used for the normalization. In another embodiment, the actual finger size can be measured.

Rather than comparing PI between different sites, which requires to consider a number of factors, the blood perfusion value presented herein is more appropriate and clinically relevant as it is a site independent measure that has been normalized based on optical path length or a surrogate such as the distance between the light emitter and the light detector (herein generally called measurement-related information) that provides a clinician with a more realistic indicator of relative perfusion at the different sites.

According to embodiments of the present invention a normalization may be applied that may be based on (a) preset values based on the sensor and site of application for a nominal optical path length that would be used to "correct" the PI value or modulation depth value, (b) an estimation of the spacing using sensors such a pressure sensor integrated into the adjustable clip sensor design or (c) a measure of the optical path length via known optical methods in order to apply the correction.

Fig. 5 shows a schematic diagram of another embodiment of a system 2 according to the present invention. In addition to the system 1 shown in Fig. 1, the system 2 comprises a distance information element 40 that is configured to measure the distance between the light emitter 11 and the light detector 12 of the sensor 10 or to determine distance-related information allowing to determine the distance between the light emitter 11 and the light detector 12. The distance information element 40 can be a separate element or can be integrated into the sensor 10 or into the device 20, depending on the way in which the distance information element 40 is implemented and how it acquires the desired information.

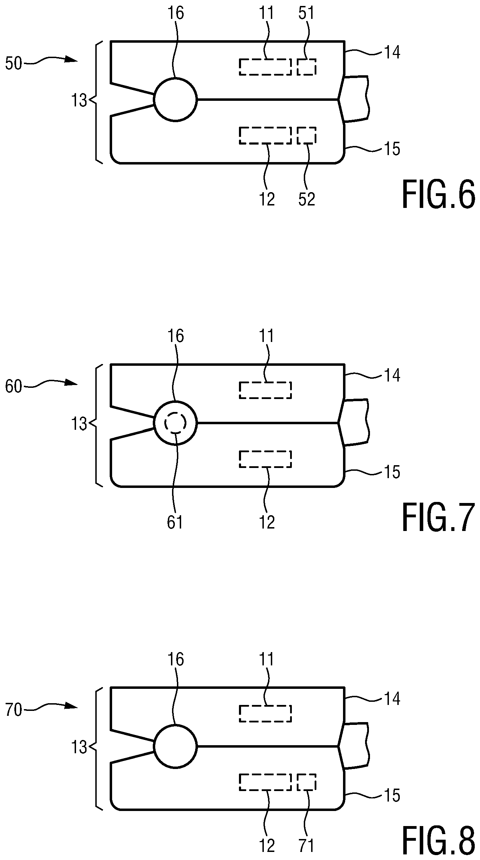

In an embodiment, the distance information element 40 comprises a distance measurement element that is configured to measure the distance or optical path length between the light emitter 11 and the light detector 12 (in the closed state in case the sensor is designed as clip as shown in Fig. 2). Fig. 6 shows an embodiment of a sensor 50 that includes, in addition to the elements of the sensor 10 shown in Fig. 2, such a distance measurement element comprising a radiation emitter 51 and a radiation detector 52. It may e.g. be configured as a time of flight (TOF) sensor that can measure a distance travelled by the light. This distance may give an estimate of the distance or path length between light emitter 11 and light detector 12. Optionally, such a sensor can simultaneously measure a PPG signal.

In another embodiment, the distance information element 40 comprises an angle measurement element that is configured to measure the angle between the light emitter and the light detector of the sensor configured as clip. Fig. 7 shows an embodiment of a sensor 60 that includes, in addition to the elements of the sensor 10 shown in Fig. 2, such an angle measurement element 61, which is integrated into the swivel joint 16 and measures the angle between the two housing portions 14, 15. The larger the angle, the larger is the distance between the light emitter 11 and the light detector 12 and thus the length of the optical path. The angle is hence a measure for the optical path length.

In another embodiment, the distance information element 40 comprises a pressure measurement element configured to measure the pressure or force applied on the anatomical object at the measurement location by the sensor configured as clip. Fig. 8 shows an embodiment of a sensor 70 that includes, in addition to the elements of the sensor 10 shown in Fig. 2, such pressure measurement element 71, which is integrated in one or both of the housing portions 14, 15. The larger the pressure, the larger is the distance between the light emitter 11 and the light detector 12 and thus the length of the optical path. The pressure is hence a measure for the optical path length.

In another embodiment, the distance information element 40 comprises a distance estimation element that is configured to estimate the distance or optical path length between the light emitter 11 and the light detector 12 based on subject-related data and/or location-related data. The distance estimation element may e.g. be integrated into the device, e.g. may be implemented by processing circuitry or software implemented on a programmed processor. The optical path length may thus be determined empirically based on available or input information, in particular about the subject and/or the sensor.

Subject-related data may include one or more of the following information (as well as its assumed influence on the distance between light emitter and light detector and thus the length of the optical path):
- the subject's body mass index (the larger the BMI, the larger the distance and path length);
- the subject's weight (the larger the weight, the larger the distance and path length);
- the subject's size (the larger the size, the larger the distance and path length);
- the subject's gender (men have typically thicker fingers so the normalization for men would assume a higher nominal distance / path length for the normalization than for women);
- the subject's race or ethnicity (ethnicities that are generally larger, e.g. people from Northern Europe are - on average - taller than people from Southern Europe, have likely also thicker fingers so they a higher nominal distance / path length would be assumed);
- the subject's health condition or diagnosis (if there is a health condition or diagnosis that is known to affect the average modulation depth, e.g. a vascular lesion on the finger, then this effect could be accounted for and thus render a better indication of the perfusion of the particular patient/patient group; for example, in a vascular lesion like port wine stains that can occur on the nose or ear, the normal measured PI of an otherwise healthy person (with a port wine stain) could be smaller due to the vascular lesion);
- the subject's physiological status;
- the subject's blood pressure;
- the subject's fluid status;
- the orthostatic position of the measurement location (when the hand is held up, the PPG modulation depth measured on fingers tends to increase; by accounting for this effect a PI value that is more representative of the actual perfusion status of the subject may be obtained); and
- the thickness of an anatomical object at the measurement location, in particular the thickness of a finger, the earlobe or the alar of the subject.

Location-related data may include one or more of the following information (as well as its assumed influence on the distance between light emitter and light detector and thus the length of the optical path):
- the location of measurement of the PPG signal (e.g. the different fingers have different thicknesses and thus lead to different distances and path lengths);
- the type of anatomical object at the measurement location (e.g. a finger generally is thicker than the ear lobe);
- the type of sensor used for acquiring the PPG signal (if the type of sensor is known, the distance between light emitter and light detector may be known);
- the temperature and/or shunt status of the tissue at the measurement location (shunt status refers to whether arterio-venous anastomoses or AVS (also known as shunts) between arterioles and venules are open or closed; to minimize heat loss the capillary system may be bypassed; the shunts may be closed to force more blood to the capillary bed if needed);
- the geometry of light transmission through an anatomical object at the measurement location from the light emitter to the light detector (the geometry may influence the distance and path length);
- the distance between the light emitter and the light detector of the sensor (the distance influences the path length and may be known or measured);
- the total attenuation of the light through the anatomical object at the measurement location from the light emitter to the light detector.

It shall be noted that in another embodiment the blood perfusion value of the subject is determined by normalizing the modulation depth or perfusion index based on measurement-related information that are indicative of the tissue volume of the subject's tissue through which the light travels from the light emitter to the light detector. The relevant volume depends on the attenuation in the anatomical object. If the attenuation is weak (e.g. when there is little blood), the volume will be larger than in the case of more blood (stronger attenuation), even with the same emitter-detector distance and the same thickness of the anatomical object at the measurement location. Even though a lot of light may travel through a substantial part of the anatomical object, e.g. across the axis of it for example, the characteristic volume that matters is the volume probed by the light that is actually detected.

As explained in the embodiments described above, the optical path or distance between the source and detector can be directly or indirectly measured/estimated or inferred with knowledge of the sensor used, the sensor site and/or the subject. A direct measurement of the optical path may be done with known optical methods. In the case of a clip design, measurement of the angle between the two portions of clip can permit an indirect estimation of the distance between source and detector to be determined. With the placement of a clip design on a known body part, the nominal thickness of that part may be used for normalization which should provide an approximate but still useful correction.

Further, as mentioned above the PI is calculated slightly differently by different manufacturers. Often, it is computed as the ratio of AC/DC components of the near-infrared PPG or a linear combination of the AC/DC components of the red and near-infrared PPG. Further, PI is often measured peripherally on the finger. The blood perfusion value introduced by the present invention is advantageous over the PI since it normalizes the PI (or modulation depth) values. Further, it may scale the PI (or modulation depth) values or the DC components from other sites based on the measured or estimated ratio of the optical path between the two sites. The normalization may be obtained by subjecting the modulation depth or perfusion index to a normalization function including one or more normalization values and determining the one or more normalization values from the measurement-related information. A look-up table or a normalization model or a linear, exponential, logarithmic or polynomial normalization function may be used for normalizing the determined modulation depth or perfusion index, in particular a normalization function that is determined empirically or by use of an analytically determined function. The obtained blood perfusion value can hence serve as a more absolute and objective measure of perfusion that allows comparison of measurements taken at different sites and/or taken for different subjects and/or with different sensors.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for determining a blood perfusion value of a subject, the device comprising circuitry configured to:
- obtain a photoplethysmography, PPG, signal acquired by a sensor including a light emitter and a light detector at a measurement location of the subject;
- determine a modulation depth of the obtained PPG signal; and
- determine the blood perfusion value of the subject by normalizing, based on measurement-related information indicative of the length of the light path of the light through the subject's tissue from the light emitter to the light detector and/or of the tissue volume of the subject's tissue through which the light travels from the light emitter to the light detector, the determined modulation depth or a perfusion index determined from the modulation depth.

2. Device according to claim 1,
wherein the circuitry is configured to
- subject the determined modulation depth or perfusion index to a normalization function including one or more normalization values and
- determine the one or more normalization values from the measurement-related information.

3. Device according to claim 2,
wherein the circuitry is configured to use a look-up table or a normalization model or a linear, exponential, logarithmic or polynomial normalization function for normalizing the determined modulation depth or perfusion index, in particular a normalization function that is determined empirically or by use of an analytically determined function.

4. Device according to any preceding claim,
wherein the circuitry is configured to use subject-related data as measurement-related information or to derive measurement-related information from subject-related data, the subject-related data including one or more of:
- the subject's body mass index,
- the subject's weight,
- the subject's size,
- the subject's gender,
- the subject's race or ethnicity,
- the subject's health condition or diagnosis,
- the subject's physiological status,
- the subject's blood pressure,
- the subject's fluid status,
- the orthostatic position of the measurement location, and
- the thickness of an anatomical object at the measurement location, in particular the thickness of a finger, the earlobe or the alar of the subject

5. Device according to any preceding claim,
wherein the circuitry is configured to use location-related data as measurement-related information or to derive measurement-related information from location-related data, the location-related data including one or more of:
- the location of measurement of the PPG signal,
- the type of anatomical object at the measurement location,
- the type of sensor used for acquiring the PPG signal,
- the temperature and/or shunt status of the tissue at the measurement location,
- the geometry of light transmission through an anatomical object at the measurement location from the light emitter to the light detector,
- the distance between the light emitter and the light detector of the sensor,
- the total attenuation of the light through the anatomical object at the measurement location from the light emitter to the light detector.

6. Device according to any preceding claim,
wherein the circuitry is configured to
- determine the distance and/or tissue volume between the light emitter and the light detector from the obtained measurement-related information, and
- normalize the determined perfusion index by multiplying it by a normalization factor that is larger the smaller the determined distance and/or the determined tissue volume is.

7. Device according to any preceding claim,
wherein the circuitry is configured to obtain the measurement-related information from a measurement element, a database or a user interface.

8. Device according to any preceding claim,
wherein the circuitry is configured to determine the perfusion index of the subject as a ratio of the AC component to the DC component of the PPG signal or of a weighted average of the AC component to the DC component of the PPG signal at different wavelengths.

9. Device according to any preceding claim,
wherein the circuitry is configured to
- determine a blood perfusion value for different PPG signals acquired at different measurement locations of the subject and/or acquired from different subjects, and
- compare and/or combine the determined blood perfusion values.

10. Device according to any preceding claim,
wherein the circuitry is configured to determine, based on the determined blood perfusion value, if the measurement location and/or arrangement of a sensor used for acquiring the PPG signal should be modified.

11. System for determining a blood perfusion value of a subject, the system comprising:
- a sensor including a light emitter and a light detector configured to acquire a photoplethysmography, PPG, signal at a measurement location of the subject; and
- a device according to any preceding claim for determining the blood perfusion value from the acquired PPG signal.

12. System according to claim 11,
wherein the system further comprises a distance information element configured to measure the distance between the light emitter and the light detector or to determine distance-related information allowing to determine the distance between the light emitter and the light detector, in particular one or more of:
- a distance measurement element configured to measure the distance or optical path length between the light emitter and the light detector,
- an angle measurement element configured to measure the angle between the light emitter and the light detector of the sensor configured as clip, and
- a distance estimation element configured to estimate the distance or optical path length between the light emitter and the light detector based on subject-related data and/or location-related data, and
- a pressure measurement element configured to measure the pressure or force applied on the anatomical object at the measurement location by the sensor configured as clip

13. Method for determining a blood perfusion value of a subject, the method comprising:
- obtaining a photoplethysmography, PPG, signal acquired by a sensor including a light emitter and a light detector at a measurement location of the subject;
- determining a modulation depth of the obtained PPG signal; and
- determining the blood perfusion value of the subject by normalizing, based on measurement-related information indicative of the length of the light path of the light through the subject's tissue from the light emitter to the light detector and/or of the tissue volume of the subject's tissue through which the light travels from the light emitter to the light detector, the determined modulation depth or a perfusion index determined from the modulation depth.

14. Computer program comprising program code means for causing a computer to carry out the steps of the method as claimed in claim 13 when said computer program is carried out on the computer.

15. Sensor for acquiring a photoplethysmography, PPG, signal at a measurement location of a subject comprising:
- a light emitter configured to emit light towards the measurement location;
- a light detector configured to detect light reflected from or transmitted through the subject's tissue at the measurement location; and
- a distance information element configured to measure the distance between the light emitter and the light detector or to determine distance-related information allowing to determine the distance between the light emitter and the light detector.
